(19) **Europäisches Patentamt**
**European Patent Office**
**Office européen des brevets**

(11) **EP 1 786 279 B1**

(12) **FASCICULE DE BREVET EUROPEEN**

(45) Date de publication et mention
de la délivrance du brevet:
**23.04.2014 Bulletin 2014/17**

(51) Int Cl.:
*C07D 233/64* (2006.01)     *A23L 1/325* (2006.01)

(21) Numéro de dépôt: **04786403.8**

(22) Date de dépôt: **19.08.2004**

(86) Numéro de dépôt international:
**PCT/IB2004/002732**

(87) Numéro de publication internationale:
**WO 2006/018673 (23.02.2006 Gazette 2006/08)**

(54) **PROCEDE DE DETERMINATION DU TAUX D'HISTAMINE DANS LES PRODUITS HALIEUTIQUES**

VERFAHREN ZUR BESTIMMUNG DES HISTAMINGEHALTS VON FISCHPRODUKTEN

METHOD FOR DETERMINING THE LEVEL OF HISTAMINE IN HALIEUTIC PRODUCTS

(84) Etats contractants désignés:
**AT BE BG CH CY CZ DE DK EE ES FI FR GB GR HU IE IT LI LU MC NL PL PT RO SE SI SK TR**

(43) Date de publication de la demande:
**23.05.2007 Bulletin 2007/21**

(73) Titulaire: **Tine, Alphonse**
**Dakar - Fann (SN)**

(72) Inventeur: **Tine, Alphonse**
**Dakar - Fann (SN)**

(74) Mandataire: **Fosse, Danièle**
**Cabinet Blétry & Associés**
**23, rue du Renard**
**75004 Paris (FR)**

(56) Documents cités:
- **DOUABALE S.E. ET AL.: "Contributions to the determination of histamine rate by measuring out the histamine-orthophthalaldehyde complex in the absorption and fluorescence." TALANTA, vol. 60, no. 2-3, 13 juin 2003 (2003-06-13), pages 581-590, XP002322194**
- **JOURNAL OFFICIEL DE LA REPUBLIQUE DE DJIBOUTI, no. 18, 30 septembre 2003 (2003-09-30), XP002322195**
- **DATABASE WPI Section Ch, Week 200357 Derwent Publications Ltd., London, GB; Class B04, AN 2003-601340 XP002322196 & JP 2003 061650 A (AICHI KEN PREFECTURE) 4 mars 2003 (2003-03-04)**
- **DATABASE WPI Section Ch, Week 200025 Derwent Publications Ltd., London, GB; Class B04, AN 2000-293137 XP002322197 & WO 00/18892 A1 (KIKKOMAN CORP) 6 avril 2000 (2000-04-06) & JP 2001 157597 A (KIKKOMAN CORP.) 12 juin 2001 (2001-06-12)**
- **DATABASE WPI Week 198652 Derwent Publications Ltd., London, GB; AN 1986-342016 XP002322198 & JP 61 254852 A (SHIMADZU SEISAKUSHO KK) 12 novembre 1986 (1986-11-12)**
- **HUNGERFORD J.M. ET AL.: "Automated kinetics-enhanced flow-injection method for histamine in regulatory laboratories: Rapid screening and suitability requirements", ANALYTICA CHIMICA ACTA, vol. 438, no. 1-2, 3 July 2001 (2001-07-03), pages 123-129,**

**Description**

L'invention porte sur un nouveau procédé de détermination du taux d'histamine, particulièrement dans les produits halieutiques, à partir de la cinétique de la formation du complexe histamine-orthophthalaldéhyde en milieu alcalin. Les produits de pêche contribuent de façon déterminante à la satisfaction des besoins alimentaires (ingrédients protéiques) de la plupart des populations mondiales. Dans certains pays qui sont producteurs, consommateurs et exportateurs de poisson, la pêche maritime occupe le premier secteur de l'économie nationale. Le nombre de personnes travaillant directement ou par induction dans le secteur de la pêche en fait un grand pourvoyeur mondial d'emplois. La pêche constitue donc dans certains pays côtiers une première source de devises, d'autant plus que la demande des produits halieutiques sur le marché mondial est en constante progression. Cependant, cette expansion s'accompagne d'une plus grande exigence des consommateurs sur la qualité sanitaire des produits. Il s'y ajoute que la plupart des grands pays importateurs ont renforcé leur réglementation sur le contrôle des aliments et deviennent de plus en plus exigeants sur les produits de pêche.

L'amélioration de la qualité sanitaire des produits de mer est devenue donc une préoccupation majeure des pouvoirs publics et de tous les acteurs dans ce domaine; particulièrement le contrôle du taux d'histamine dans les produits halieutiques. L'histamine, considérée comme médiateur chimique auprès de l'adrénaline, joue un rôle important dans le système nerveux. Cependant, ce produit est fortement toxique. L'absorption d'une certaine quantité d'histamine peut être à l'origine d'une réaction allergique provoquant des maux d'estomac, des nausées, des vomissements, des maux de têtes, des étourdissements, des démangeaisons, en passant par les rougeurs et les enflures du visage. Ces symptômes peuvent apparaître immédiatement ou plusieurs heures après l'ingestion d'aliments à teneur élevée d'histamine. La durée de ces symptômes peut aller de quelques heures à plusieurs jours dans certains cas. Pire, les conséquences d'une absorption de l'histamine peuvent être très graves pour l'homme (vasodilatation, augmentation de la perméabilité capillaire) et conduire à la mort.

Les travaux de KIM, LOPEZ-SABATER, LERKE, et de YOSHINAGA et FRANK ont montré que c'est après une exposition post-mortem que les produits halieutiques contiennent un important taux d'histamine dans leurs tissus, surtout à des températures modérément élevées. Les travaux de YOSHINAGA confirmés par ceux de OLLEY et BARANOWSKI ont montré aussi que la teneur moyenne d'histamine est maximale entre les températures de 20 et 30°C : intervalle de températures couvrant largement la température moyenne observée dans beaucoup de pays producteurs de poisson. Ces températures ambiantes favorisent l'activité des bactéries histaminogènes. Elles ont la particularité de disposer de l'histidine décarboxylase, enzyme qui catalyse la transformation de l'histidine (acide aminé naturel) en histamine. On pourrait citer parmi ces bactéries, Proteus morganii, Morganella morganii, Klebsiella pneumoniae, Klebsiella oxycota, Clostridium perfringens, Hafnia alvei, Photobacterium phosphorium, Citrobacter freundii, Enterobacter aerogenes. Parmi les familles de poissons porteurs de ces bactéries, présentant ainsi une forte propension à l'intoxication par l'histamine, on peut citer les Scombridés, Scombérésocidés, Clupéidés, Coryphénidés, Pomatomidés, Carangidés, Englaulidés, Xiphyidés, Istiophoridés. On sait également qu'à des températures moyennes, à la période post-mortem les produits halieutiques sains sont rapidement dégradables par simple contact des espèces contaminées bactériologiquement. En effet, dans un organisme vivant, tout excès d'histamine est éliminé par les actions de deux enzymes : la diamine oxydase (DAO) et l'histamine méthyltransférase (HMT). La première oxyde l'histamine en acide imidazolacétique tandis que la seconde en assure la méthylation en méthylhistamine, conduisant ainsi aux métabolites non nocifs.

Dès lors, il importe de disposer d'une méthode de dosage d'histamine capable de fournir des résultats fiables avec une limite de détection très basse. Ainsi, depuis la découverte de la molécule d'histamine au début du siècle passé (1916) par GUGGENHEIM et LOFFLER, plusieurs techniques relatives à son dosage ont été proposées. Malheureusement divers problèmes y sont associés. Les problèmes rencontrés dans les méthodes sont de plusieurs ordres et dépendent de la méthode utilisée.

La chromatographie sur couche mince présente une limite de détection trop élevée (20-50 ppm). On la qualifie alors de méthode semi-quantitative.

Les méthodes biologiques sont également peu quantitatives. Elles utilisent par exemple la contraction des fibres musculaires d'un animal très peu sensible à l'histamine. Elles nécessitent des animaux d'expérience (cobayes et daphnies).

Les méthodes radioenzymatiques emploient des radio-isotopes dont la manipulation est très minutieuse.

Les méthodes enzymatiques sont très longues à cause des durées d'incubation et des extractions multiples. Les enzymes sont peu disponibles.

Les méthodes immunologiques nécessitent des anticorps et des enzymes. Des temps d'incubation peuvent être très longs.

La spectrophotométrie par mesure de densité optique est peu quantitative. Elle comporte des risques de résultats erronés.

Les méthodes électrochimiques nécessitent des électrodes à sonde d'oxygène, mais également un enzyme qui n'est pas commercialement disponible.

**EP 1 786 279 B1**

La chromatographie liquide haute performance pose un problème de quantification lorsque la dérivatisation est réalisée en pré-colonne. Une dérivatisation post-colonne génère des variations à causes des pompes.

Les méthodes fluorimétriques en vigueur : celle de Lerke et Bell et celle de l'association officielle de chimie analytique (OAAC) sont très semblables dans la pratique. La différence entre les deux méthodes réside surtout au niveau des solvants d'extraction. La première utilise une solution d'acide trichloracétique (TCA) 10% tandis que la seconde utilise du méthanol à 75%. L'histamine est éluée d'une colonne chromatographique échangeuse d'ions par de l'acide chlorhydrique. La condensation de l'histamine avec l'orthophthalaldéhyde (OPA) est réalisée en milieu basique pour obtenir un complexe très fluorescent en milieu basique mais instable en ce milieu. C'est pourquoi le dosage par fluorescence a été fait en milieu acide. En réalités, les avantages des méthodes fluorimétriques sont associés à leur spécificité. Pour qu'il ait interférencz entre deux composés, il faudrait qu'ils présentent une même longueur d'onde d'absorption, une même longueur d'excitation et une même longueur d'onde d'émission. Un autre avantage des méthodes fluorimétriques est leur sensibilité qui permet d'obtenir des limites de détection très basses. Ainsi, par rapport aux autres méthodes existant, le dosage par voie fluorimétrique est de loin la méthode la plus utilisée car plus sensible. Cependant, des inconvénients majeurs résultent du fait que :

- Les bases de mesure ont été effectuées par un fluorimètre mais non par un spectrofluorimètre.
- La cinétique de la formation du complexe histamine-OPA en milieu basique ne peut être obtenue par un fluorimètre.
- Le milieu acide dans lequel la fluorescence est mesurée s'est révélé comme un inhibiteur de l'intensité de fluorescence du complexe. Cela a pour conséquence la réduction du domaine de calibration. Les résultats obtenus deviennent ainsi moins fiables d'autant plus que la différence entre deux concentrations distinctes est moins nette.
- On observe en milieu acide l'existence de deux types de complexes, nettement décelables par spectrofluorimètre, non par un fluorimètre même couplé à un HPLC par exemple. Donc, l'utilisation du simple fluorimètre ne permet pas la visualisation des spectres et par conséquent la mise en évidence de l'apparition du second complexe en milieu acide. Le milieu réactionnel n'est pas optimisé en fonction des concentrations d'OPA et du pH. La température réactionnelle n'est pas optimisée. Les résultats statistiques (limites de détection et taux de recouvrement) sont peu probants en milieu acide. Ces méthodes ne sont pas pratiques pour la détermination de l'histamine dans le milieu médical (sur l'homme) où le taux est généralement faible.

La recherche sur l'état de la technique au niveau des publications spécifiques a permis d'identifier les références bibliographiques ci-après, en rapport avec l'objet de l'invention :

- H.A. Frank, D.H. Yoshinaga, and W.K. Nip, Histamine formation and honeycombing during decomposition of skipjack tuna, Katsuwonus pelamis, at elevated temperature; Marine Fisheries Review; Vol.43 N° 10 (1981) 9-14.
- P.A. Lerke, S. Werne, S. Taylor, West. I. Med. 129 (1978) 381.
- SH. Kim, KG. Field, MT. Morrissey, RJ. Price, CI. Wie, AN. Haejung; Source and identification of histamine-producing bacteria from fresh and temperature-abused albacore; J. Food Protection; Vol. 64, N° 7 (2001) 1035-1044.
- P.K. Vijayan, P.K. Surendran, K.K. Balachandran, (Symp. On Tropical Marine Living Ressources, Cochin, India, Jan.12-16, 1988), J. Mar. Biol. Assoc. India 31 (1989) 202.
- C.E.M. Van Gelderen, T.J.F. Savelkoul, L.A. Van Ginkel, W. Van Dokkum, J. Toxicol. Clin. Toxicol. 30 (1992) 585.
- S.L. Taylor, J.E. Stratton, J.A. Nordlee, J. Toxicol. 27 (1989) 225
- P.A. Shore, A. Burkhalter, V.H. Jr Cohn, J. Pharmacol. Exp. Ther. 127 (1959) 182.
- S. Taylor, Histamine food poisoning: toxicity and clinical aspects, critical Rev. in Tox., (1986) 91-128.
- W. Lorenz, E. Neugebauer, B. Uvnäs, M.A. Beaven, M. Ennis, G. Granerus, J.P. Green, J.J. Keyzer, P.T. Mc Bride, P.F. Mannaioni, F.L. Pearce, J. Watkins, K. MUNICH Consensus Development Conference On Histamine Determination, Current techniques of histamine determination, 2 (1990) 81
- P.L. Rogers, W.F. Staruszkiewicz, Histamine Test Kit Comparison, J. Aquatic Food Product Technology, Vol. 9 N° 2 (2000) 5-17.
- E.I. Lopez-Sabater, J.J. Rodriguez-Jerez, A.X. Roig-Sagues, M.A.T. Mora-Ventura, Bacteriological quality of tuna fish (Thunnus Thunnus) destined for canning : effect of tuna handling on presence of histidine decarboxylase bacteria and histamine level, J. Food-Prot., 57 (4) (1994) 318-323.
- K.V. Basavakumar, I. Karunasagar, Ability of fish associated bacteria to produce amines suspected to be involved in scombroid poisoning ; Indian J. Microbiol, 32 (1) (1992) 75-79.
- C. den Brinker, C. Rayner, and M. Kerr, Investigatione of biogenic amines in fermented fish and fish products; Health Division, Victorian Gov. Dept. of Human services, Ed. 1 (2002).
- J. Olley, J. Baranowski, Temperature effects on histamine formation. In (B.P. Pan et D. Pan eds.) Histamine in marine products: production by bacteria, measurment and prediction of formation. Rome, Italie: FAO Fisheries Technical Paper 252 (1985) 14-17.

La recherche sur les brevets a donné les résultats suivants :

- Method of detecting and quantifying endotoxins. N° US2004029104.
- Fluorescent enzyme assay methods and compositions. N° WO2004022773.
- Mesuring esterase activity using fluorescent substrates as a way of evaluating cervical cancer. N° US6696241.
- Discrimination of cells using chemical characteristics. N° US2004029103.

Les documents résultants de l'état de la technique ci-dessus n'ont pas donné de solutions aux problèmes identifiés, notamment les limites de détections élevées, de très longues durées de mesure...

Le nouveau procédé de détermination du taux d'histamine dans les poissons post-mortem par la formation du complexe Histamine-orthophthalaldéhyde en milieu basique permet de donner un taux probant de l'histamine dans les produits halieutiques.

Le nouveau procédé consiste à extraire, à purifier l'histamine et à procéder à la formation du complexe entre l'histamine et l'OPA selon la méthode officielle (OAAC). Le suivi de la cinétique de formation du complexe se fait en milieu alcalin avec optimisation du milieu. Un petit volume de l'éluat d'histamine (2 mL) est mélangé avec des solutions de NaOH et d'OPA, dans un récipient contenant un petit barreau aimanté et soumis à une agitation dans les conditions optimales : OPA ($10^{-5}$-$10^{-4}$ M) / NaCl (0 - 0,2 M) / pH 11,48. Au bout de 3 minutes de réaction à 50°C, 2,5 mL du mélange dans les conditions optimales sont introduits dans une cuve en quartz et placés dans le spectrofluorimètre. La courbe de cinétique est lancée pour une durée de 10minutes à la température ambiante. L'intensité maximale du palier de cinétique correspondant au mélange réactionnel est relevée. A partir des intensités maximales correspondant aux paliers de la cinétique, une courbe de calibration est déterminée. Une extrapolation sur cette courbe permet d'en déduire la concentration en histamine. Le taux d'histamine de l'échantillon est déterminé en remontant par le calcul correctif des diverses dilutions.

$$\text{Taux} = \frac{V_{ext} \bullet V_{rea} \bullet V_{col} \bullet 10^{-3} \bullet 111,1 \bullet 10^{6} \bullet [\text{Histamine}]}{V_{fil} \bullet V_{elu} \bullet m} \qquad \text{(ppm)}$$

Où V = volume (mL) ; ext = extraction ; fil = filtrat prélevé; elu = éluat prélevé ; rea = réactionnel ; col = total éluat de la colonne ; m = masse de l'échantillon (g), [Histamine] = concentration d'histamine dans la cuve (mol.$L^{-1}$).

Le nouveau procédé de détermination du taux d'histamine à partir de la cinétique de la formation du complexe OPA-Histamine en milieu basique, où l'intensité de la fluorescence est très intense, a permis de résoudre les problèmes suivants : le domaine de calibration plus élevé, la limite de détection plus basse, les taux de recouvrement proches de 100%, la fiabilité des mesures plus probante, la possibilité de stabiliser le complexe en milieu basique.

De même, le nouveau procédé a également permis d'observer les phénomènes ci-après :

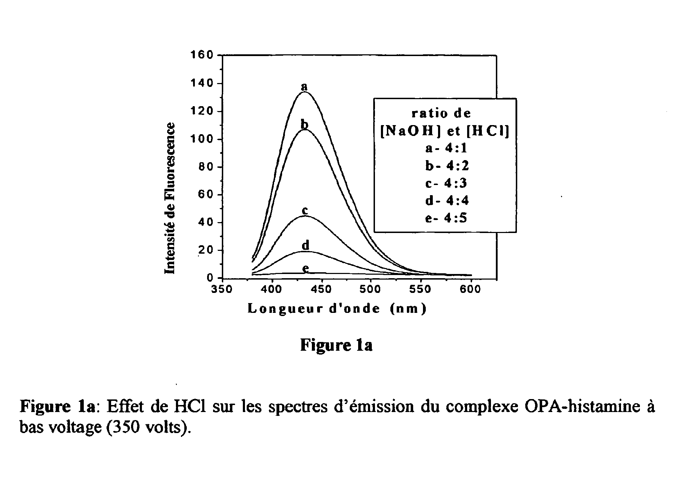

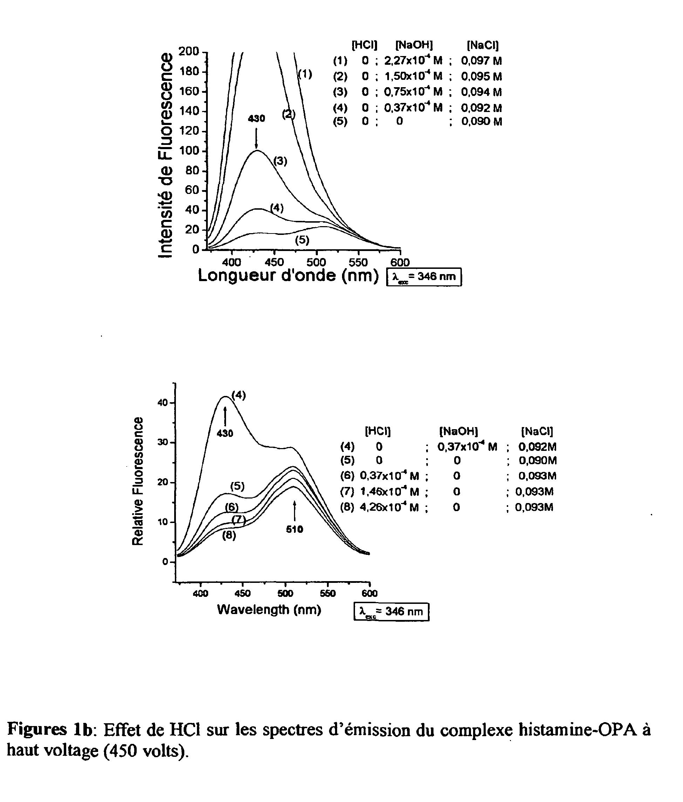

- L'existence de 2 types de complexes en milieu acide : l'acide chlorhydrique inhibe la fluorescence du complexe OPA-Histamine **(figure 1a)**. Cependant, quand on augmente l'énergie d'excitation (400 V), en milieu acide, on décèle deux types de complexes, l'un émettant aux environs de 430 nm et l'autre émettant aux environs de 510 nm **(figure 1b)**. Par conséquent, non seulement l'acide chlorhydrique (HCl) inhibe la fluorescence, mais favorise la formation de deux types de complexe entre l'histamine et l'OPA. Dès lors, une étude quantitative dans ce milieu pour déterminer le taux d'histamine pose problème. C'est pourquoi nous avons envisagé de travailler en milieu basique en observant la cinétique de la formation du complexe OPA-Histamine où l'intensité de la fluorescence est très intense.

- Optimisation des concentrations de NaOH, de NaCl et effet de température :

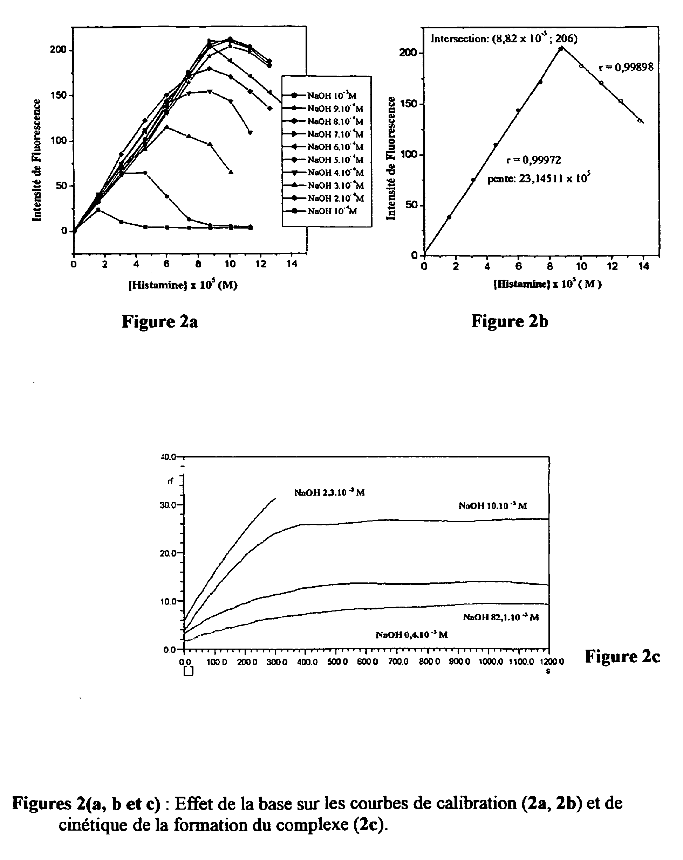

  * Les résultats obtenus sur les courbes de calibration en milieu basique indiquent une extension du domaine de calibration avec l'augmentation de la concentration de NaOH jusqu'aux valeurs proches de $10^{-3}$ M **(figures 2a** et **2b)**. Le maximum du domaine de calibration se situe aux environs du pH 11,5, au-delà, on constate un effet inhibiteur de NaOH. Le même phénomène est observé sur les maxima de la cinétique de formation du complexe en fonction de la concentration de NaOH **(figure 2c).**

  * L'étude de l'effet de la variation de concentration de chlorure de sodium (NaCl) entre 0 et 0,6 M a montré que NaCl n'a pas d'influence sur l'intensité de fluorescence du complexe OPA-Histamine.

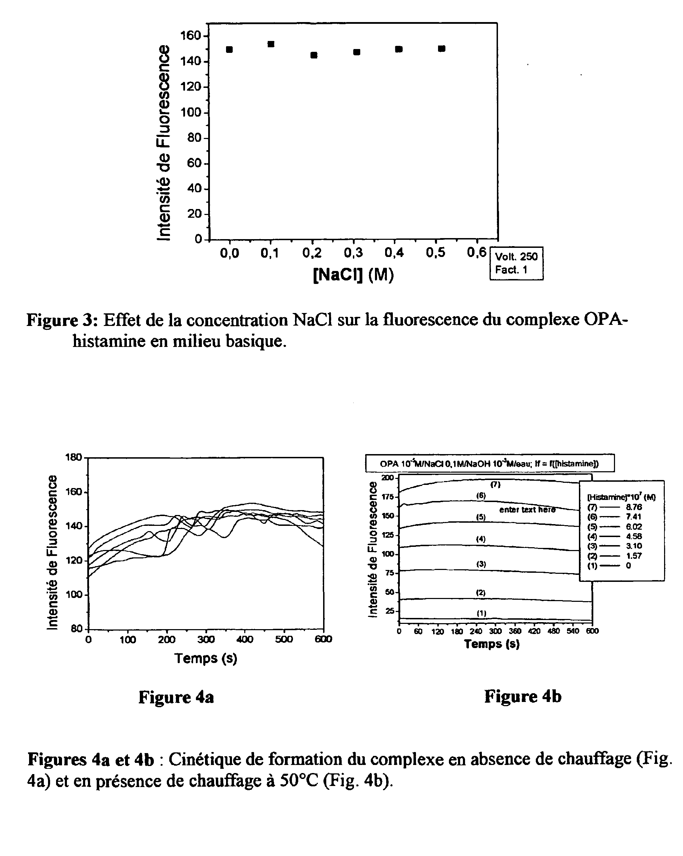

  * L'une des principales difficultés rencontrées par les méthodes connues dans le dosage de l'histamine est l'instabilité du complexe qu'elle forme avec l'orthophthalaldéhyde, en milieu basique. Pour contourner cette difficulté, la cinétique de la formation du complexe OPA-Histamine en milieu basique **(pH 11,5)** a été étudiée. Cette cinétique admet un maximum correspondant au temps $t_{max}$. Cependant, si le chauffage n'a pas lieu, il est très difficile de déterminer le maximum puisque les courbes présentent des ondulations non négligeables **(figure 4a).** Cette figure montre aussi que le temps de formation du complexe ($t_{max}$) est relativement long (aux environs de 5 minutes). Dans le milieu optimal (OPA ($10^{-5}$ - $10^{-4}$ M) / NaCl (0 - 0,2 M) / pH 11,48) chauffé à 50°C pendant 3 minutes, on parvient à avoir des courbes de cinétique relativement stables et un temps de formation du complexe relativement court ($t_{max}$ compris en général entre 2 et 3 minutes) **(figure 4b),** ce qui permet de mesurer avec précision le taux d'histamine, avec un domaine de calibration très large ($I_f$ compris entre 0 et 100). Si la température de chauffage est à 50°C, la cinétique observée et toutes les mesures ont été faites à la température ambiante.

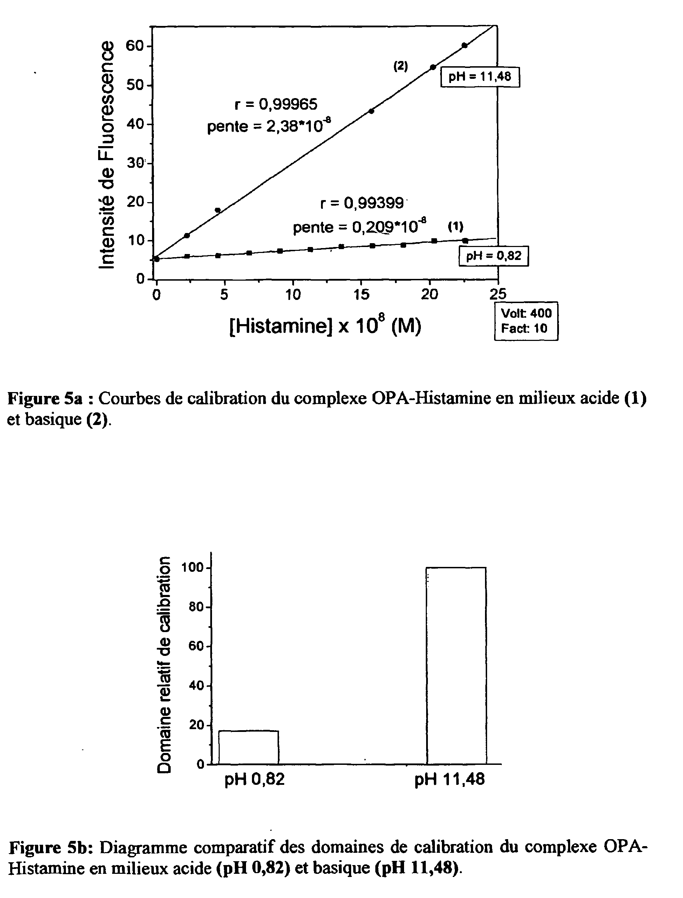

- Les courbes de calibration sont obtenues à partir des maxima de la cinétique de formation du complexe OPA-Histamine en milieu basique. En milieu acide, les courbes de calibration sont obtenues à partir des intensités de fluorescence du complexe OPA-Histamine. Les coefficients de corrélation obtenus sont très proches de l'unité dans les deux cas, 0,993 en milieu acide et 0,999 en milieu basique. Cependant, la pente de la droite de calibration et le domaine de calibration sont dix fois plus élevés en milieu basique qu'en milieu acide **(figures 5a, 5b).** Ce domaine réduit en milieu acide montre le caractère inhibiteur de l'acide chlorhydrique sur l'intensité de fluorescence du complexe OPA-Histamine.

- Les résultats statistiques sont résumés dans le tableau ci-après. Ces résultats montrent que les coefficients de corrélation sont supérieurs à 0,999 en milieu basique au lieu de 0,993 en milieu acide, ce qui indique la précision des mesures. Par ailleurs, la comparaison des résultats statistiques obtenus dans les deux milieux (basique et acide) montre que :

  * Le domaine de linéarité est au moins 10 fois plus élevé en milieu basique qu'en milieu acide **(voir figures 5a et 5b).**
  * La limite de détection est 10 fois plus faible par le nouveau procédé d'invention qu'en milieu acide.
  * Le nouveau procédé est beaucoup plus précis et plus fiable au regard des taux de recouvrement compris entre 89 et 114% au lieu de 17 à 217%. Ce mauvais taux de recouvrement observé en milieu acide est normal car l'acide chlorhydrique inhibe la fluorescence du complexe formé. En plus, le complexe émettant à 430 nm est en compétition avec un autre qui émet à 510 nm en milieu HCl.

**Tableau : Comparaison des résultats statistiques obtenus dans les deux milieux**

| Méthode | Domaine relatif de calibration en If | Limite de détection (ng/ml) | Taux de recouvrement | Coefficients de corrélation |
|---|---|---|---|---|
| Nouveau procédé milieu basique | 1-100 | 0,25 | 89% - 114% | 0,9996 |
| Méthode en milieu acide | 0-10 | 2,5 | 17% - 217% | 0,9940 |

**Revendications**

1. Procédé de détermination du taux d'histamine dans des produits halieutiques à partir d'une cinétique de formation d'un complexe histamine-orthophtalaldéhyde en milieu alcalin par voie spectrofluorimétrique, comprenant des étapes de

   - purification de l'histamine,
   - complexation de l'histamine à l'OPA dans un milieu optimal OPA ($10^{-5}$-$10^{-4}$ M)/NaCl (0-0,2 M)/ pH 11,48),
   - observation de la cinétique de la formation du complexe OPA- Histamine en milieu alcalin pour déterminer le taux d'histamine,

   **caractérisé en ce que** l'étape de complexation comprend un chauffage du milieu optimal à 50°C pendant au plus 600 s, de préférence pendant 3 minutes.

2. Procédé de détermination du taux d'histamine dans les produits halieutiques selon la revendication 1, **caractérisé en ce que** la sous-étape de chauffage est réalisée pendant 3 minutes à 50°C.

3. Procédé de détermination du taux d'histamine selon l'une des revendications précédentes, **caractérisé en ce que** des courbes de calibration sont déterminées à partir des intensités maximales de fluorescence de la cinétique de formation du complexe OPA-histamine.

4. Procédé de détermination du taux d'histamine dans les produits halieutiques selon l'une des revendications précédentes, **caractérisé en ce que** le taux d'histamine de l'échantillon est déterminé par la lecture des intensités maximales correspondant aux paliers de la cinétique.

5. Procédé de détermination du taux d'histamine selon l'une des revendications précédentes, **caractérisé en ce que** les courbes de calibration sont déterminées à partir des intensités maximales de fluorescence OPA-histamine dans une solution tampon de pH comprise entre 11,45 et 11,5.

**Patentansprüche**

1. Verfahren zur Bestimmung des Histamingehalts in Fischprodukten, ausgehend von einer Kinetik der Bildung eines Ortho-Phtalaldhyd-Histamin-Komplexes in alkalinem Medium auf spektrofluorometrischem Weg, umfassend die folgenden Schritte:

  - Reinigen des Histamins,
  - Komplexbilden des Histamins an OPA in einem optimalen OPA-Medium ($10^{-5}$-$10^{-4}$ M)/NaCl (0-0,2 M)/ pH 11,48),
  - Beobachten der Kinetik der Bildung des Komplexes OPA-Histamin in alkalinem Medium, um den Histamingehalt zu bestimmen,

  **dadurch gekennzeichnet, dass** der Schritt des Komplexbildens eine Erhitzung des optimalen Mediums auf 50 °C während höchstens 600 s, vorzugsweise während 3 Minuten umfasst.

2. Verfahren zur Bestimmung des Histamingehalts in Fischprodukten nach Anspruch 1, **dadurch gekennzeichnet, dass** der Unterschritt des Erhitzens während 3 Minuten bei 50°C durchgeführt wird.

3. Verfahren zur Bestimmung des Histamingehalts nach einem der vorhergehenden Ansprüche, **dadurch gekennzeichnet, dass** Kalibrierungskurven ausgehend von den maximalen Fluoreszenzintensitäten der Kinetik der Bildung des Komplexes OPA-Histamin bestimmt werden.

4. Verfahren zur Bestimmung des Histamingehalts in Fischprodukten nach einem der vorhergehenden Ansprüche, **dadurch gekennzeichnet, dass** der Histamingehalt der Probe durch das Ablesen der maximalen Intensitäten bestimmt wird, die den Kinetiklagern entsprechen.

5. Verfahren zur Bestimmung des Histamingehalts nach einem der vorhergehenden Ansprüche, **dadurch gekennzeichnet, dass** die Kalibrierungskurven ausgehend von den maximalen Fluoreszenzintensitäten von OPA-Histamin in einer Pufferlösung mit einem pH-Wert zwischen 11,45 und 11,5 bestimmt werden.

**Claims**

1. Method for determining the level of histamine in halieutic products on the basis of histamine-orthophthaldehyde complex formation kinetics in an alkaline medium by means of a spectrofluorimetric process, comprising steps for

  - purifying histamine,
  - complexing histamine with OPA in an optimal OPA ($10^{-5}$-$10^{-4}$ M)/NaCl (0-0.2 M)/pH 11.48) medium,
  - observing the OPA-Histamine complex formation kinetics in an alkaline medium to determine the level of histamine,

  **characterised in that** the complexing step comprises heating the optimal medium to 50°C for not more than 600 s, preferably for 3 minutes.

2. Method for determining the level of histamine in halieutic products according to claim 1, **characterised in that** the heating sub-step is performed for 3 minutes at 50°C.

3.  Method for determining the level of histamine according to any of the above claims, **characterised in that** the calibration curves are determined on the basis of the maximum fluorescence intensities of the OPA-histamine complex formation kinetics.

4.  Method for determining the level of histamine in halieutic products according to any of the above claims, **characterised in that** the level of histamine of the sample is determined by reading the maximum intensities corresponding to the stages of the kinetics.

5.  Method for determining the level of histamine according to any of the above claims, **characterised in that** the calibration curves are determined on the basis of the maximum OPA-histamine fluorescence intensities in a buffer solution having a pH between 11.45 and 11.5.

**Figure 1a**

**Figure 1a**: Effet de HCl sur les spectres d'émission du complexe OPA-histamine à bas voltage (350 volts).

|  | [HCl] | [NaOH] | [NaCl] |
|---|---|---|---|
| (1) | 0 : | $2,27 \times 10^{-4}$ M ; | 0,097 M |
| (2) | 0 : | $1,50 \times 10^{-4}$ M ; | 0,095 M |
| (3) | 0 : | $0,75 \times 10^{-4}$ M ; | 0,094 M |
| (4) | 0 : | $0,37 \times 10^{-4}$ M ; | 0,092 M |
| (5) | 0 : | 0 ; | 0,090 M |

$\lambda_{exc}$ = 346 nm

|  | [HCl] | [NaOH] | [NaCl] |
|---|---|---|---|
| (4) | 0 ; | $0,37 \times 10^{-4}$ M ; | 0,092M |
| (5) | 0 ; | 0 ; | 0,090M |
| (6) | $0,37 \times 10^{-4}$ M ; | 0 ; | 0,093M |
| (7) | $1,46 \times 10^{-4}$ M ; | 0 ; | 0,093M |
| (8) | $4,26 \times 10^{-4}$ M ; | 0 ; | 0,093M |

$\lambda_{exc}$ = 346 nm

**Figures 1b**: Effet de HCl sur les spectres d'émission du complexe histamine-OPA à haut voltage (450 volts).

**Figure 2a**

**Figure 2b**

**Figure 2c**

**Figures 2(a, b et c)** : Effet de la base sur les courbes de calibration (**2a, 2b**) et de cinétique de la formation du complexe (**2c**).

**Figure 3:** Effet de la concentration NaCl sur la fluorescence du complexe OPA-histamine en milieu basique.

**Figure 4a**

**Figure 4b**

**Figures 4a et 4b** : Cinétique de formation du complexe en absence de chauffage (Fig. 4a) et en présence de chauffage à 50°C (Fig. 4b).

**Figure 5a** : Courbes de calibration du complexe OPA-Histamine en milieux acide (1) et basique (2).

**Figure 5b:** Diagramme comparatif des domaines de calibration du complexe OPA-Histamine en milieux acide (**pH 0,82**) et basique (**pH 11,48**).

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2004029104 A
- WO 2004022773 A
- US 6696241 B
- US 2004029103 A

**Littérature non-brevet citée dans la description**

- **H.A. FRANK ; D.H. YOSHINAGA ; W.K. NIP.** Histamine formation and honeycombing during decomposition of skipjack tuna, Katsuwonus pelamis, at elevated temperature. *Marine Fisheries Review,* 1981, vol. 43 (10), 9-14
- **P.A. LERKE ; S. WERNE ; S. TAYLOR.** *West. I. Med.,* 1978, vol. 129, 381
- **SH. KIM ; KG. FIELD ; MT. MORRISSEY ; RJ. PRICE ; CI. WIE.** AN. Haejung; Source and identification of histamine-producing bacteria from fresh and temperature-abused albacore. *J. Food Protection,* 2001, vol. 64 (7), 1035-1044
- **P.K. VIJAYAN ; P.K. SURENDRAN ; K.K. BALACHANDRAN.** *Symp. On Tropical Marine Living Ressources, Cochin, India,* 12 Janvier 1988
- *J. Mar. Biol. Assoc. India,* 1989, vol. 31, 202
- **C.E.M. VAN GELDEREN ; T.J.F. SAVELKOUL ; L.A. VAN GINKEL ; W. VAN DOKKUM.** *J. Toxicol. Clin. Toxicol.,* 1992, vol. 30, 585
- **S.L. TAYLOR ; J.E. STRATTON ; J.A. NORDLEE.** *J. Toxicol.,* 1989, vol. 27, 225
- **P.A. SHORE ; A. BURKHALTER ; V.H. JR COHN.** *J. Pharmacol. Exp. Ther.,* 1959, vol. 127, 182
- **S. TAYLOR.** Histamine food poisoning: toxicity and clinical aspects, critical Rev. *Tox.,* 1986, 91-128
- **W. LORENZ ; E. NEUGEBAUER ; B. UVNÄS ; M.A. BEAVEN ; M. ENNIS ; G. GRANERUS ; J.P. GREEN ; J.J. KEYZER ; P.T. MC BRIDE ; P.F. MANNAIONI.** *Consensus Development Conference On Histamine Determination, Current techniques of histamine determination,* 1990, vol. 2, 81
- **P.L. ROGERS ; W.F. STARUSZKIEWICZ.** Histamine Test Kit Comparison. *J. Aquatic Food Product Technology,* 2000, vol. 9 (2), 5-17
- **E.I. LOPEZ-SABATER ; J.J. RODRIGUEZ-JEREZ ; A.X. ROIG-SAGUES ; M.A.T. MORA-VENTURA.** Bacteriological quality of tuna fish (Thunnus Thunnus) destined for canning : effect of tuna handling on presence of histidine decarboxylase bacteria and histamine level. *J. Food-Prot.,* 1994, vol. 57 (4), 318-323
- **K.V. BASAVAKUMAR ; I. KARUNASAGAR.** Ability of fish associated bacteria to produce amines suspected to be involved in scombroid poisoning. *Indian J. Microbiol,* 1992, vol. 32 (1), 75-79
- Investigatione of biogenic amines in fermented fish and fish products. **C. DEN BRINKER ; C. RAYNER ; M. KERR.** Health Division. 2002
- Temperature effects on histamine formation. **J. OLLEY ; J. BARANOWSKI.** Histamine in marine products: production by bacteria, measurment and prediction of formation. Rome, Italie. 1985, vol. 252, 14-17